# EUROPEAN PATENT SPECIFICATION

(11) **EP 3 947 671 B1**
(45) Date of publication and mention of the grant of the patent: **17.09.2025**
(21) Application number: 20722021.1
(22) Date of filing: 31.03.2020
(51) Int. Cl.: C12N 15/10

(54) **METHODS OF SELECTING AND DETECTING BINDING PEPTIDES**
VERFAHREN ZUR AUSWAHL UND DETEKTION VON BINDUNGSPEPTIDEN
PROCÉDÉS DE SÉLECTION ET DE DÉTECTION DE PEPTIDES DE LIAISON

(30) Priority: 02.04.2019 US 201962828040 P
(43) Date of publication of application: 09.02.2022
(73) Proprietor: Eli Lilly and Company, Indianapolis, IN 46285 (US)
(72) Inventor: AFSHAR, Sepideh, Indianapolis, Indiana 46206-6288 (US)
(74) Representative: Eli Lilly and Company Limited
(86) International application number: PCT/US2020/025915
(87) International publication number: WO 2020/205836

(56) References cited:
- WO-A1-2016/134521
- JUNICHI YAMAGUCHI ET AL: "cDNA display: a novel screening method for functional disulfide-rich peptides by solid-phase synthesis and stabilization of mRNA?protein fusions", NUCLEIC ACIDS RESEARCH, OXFORD UNIVERSITY PRESS, GB, vol. 37, no. 16, 1 September 2009 (2009-09-01), pages e108 - 1, XP008154704, ISSN: 0305-1048, [retrieved on 20090615], DOI: 10.1093/NAR/GKP514
- FARZAD JALALI-YAZDI ET AL: "Robust, Quantitative Analysis of Proteins using Peptide Immunoreagents, in Vitro Translation, and an Ultrasensitive Acoustic Resonant Sensor", ANALYTICAL CHEMISTRY, vol. 86, no. 10, 20 May 2014 (2014-05-20), US, pages 4715 - 4722, XP055364635, ISSN: 0003-2700, DOI: 10.1021/ac500084d
- YU NAGUMO ET AL: "PURE mRNA display for in vitro selection of single-chain antibodies", JOURNAL OF BIOCHEMISTRY, vol. 159, no. 5, 28 December 2015 (2015-12-28), GB, pages 519 - 526, XP055693743, ISSN: 0021-924X, DOI: 10.1093/jb/mvv131
- YUKI MOCHIZUKI ET AL: "One-Pot Preparation of mRNA/cDNA Display by a Novel and Versatile Puromycin-Linker DNA", ACS COMBINATORIAL SCIENCE, vol. 13, no. 5, 12 September 2011 (2011-09-12), US, pages 478 - 485, XP055277429, ISSN: 2156-8952, DOI: 10.1021/co2000295
- SHINGO UENO ET AL: "Improvement of a puromycin-linker to extend the selection target varieties in cDNA display method", JOURNAL OF BIOTECHNOLOGY, vol. 162, no. 2-3, 19 September 2012 (2012-09-19), AMSTERDAM, NL, pages 299 - 302, XP055277425, ISSN: 0168-1656, DOI: 10.1016/j.jbiotec.2012.09.003
- NORD O ET AL: "Microbead display of proteins by cell-free expression of anchored DNA", JOURNAL OF BIOTECHNOLOGY, ELSEVIER, AMSTERDAM, NL, vol. 106, no. 1, 5 December 2003 (2003-12-05), pages 1 - 13, XP002539802, ISSN: 0168-1656, DOI: 10.1016/J.JBIOTEC.2003.09.002

## Description

The present invention is in the field of mRNA display technologies. More particularly, the present invention relates to methods for selecting, detecting, and/or enriching peptides against cell-expressed or soluble peptides and peptides with desired functional properties.

Messenger RNA (mRNA) display is a selection technique against soluble peptides and soluble proteins. (Szostak R. et al., RNA-peptide fusions for the in vitro selection of peptides and proteins. Proc. Natl. Acad. Sci. 94, 12297-12302 (1997)). Messenger RNA display enables construction of highly diverse peptide libraries that may include natural and non-natural amino acids. Incorporation of non-natural amino acids is made possible by different approaches including suppression of amber codon and utilization of modified tRNA (chemical conjugation of non-natural amino acid to tRNA) or by a highly flexible tRNA aminoacylation enzyme made of artificial ribozymes, called flexizymes (Niwa N. et al., A flexizyme that selectively charges amino acids activated by a water-friendly leaving group. Bioorganic & Med. Chem. Letters 19(14), 3892-3894 (2009)).

The process of selecting peptides with mRNA display traditionally includes transcribing mRNA from a variant pool of DNA and annealing the mRNA variants to a short DNA linker that is conjugated to puromycin at its 3'-end (Ishizawa T. et al., TRAP Display: A High-Speed Selection Method for the Generation of Functional Polypeptides. J. Am. Chem. Soc. 135(14), 5433-5440 (2013)). This creates an mRNA:DNA linker-puromycin library. Translation is initiated by adding bacterial or rabbit reticulocyte lysate or Pure Express^{®}.

During translation, ribosomes move along individual mRNA and translate the genetic information stored in mRNA into the peptide sequence. When ribosomes encounter a TAG stop codon engineered at the end of the peptide sequence, they stall in the absence of release factor 1, allowing puromycin to enter the ribosome site-A. As a result, puromycin makes a covalent linkage between the polypeptide growing chain and the mRNA. Translation is halted, and the mRNA that is hybridized to the DNA linker fused to puromycin is now linked to the peptide. The mRNA is then reverse transcribed to cDNA to allow for PCR amplification and to generate a stable DNA:RNA duplex with reduced nonspecific binding during selection. The library is selected against a target of interest, non-specific peptides are washed away, and binders are eluted. Selection efficiency is monitored by quantitative PCR (qPCR). Next generation sequencing is used to determine peptide sequences that are enriched after multiple rounds of sequences.

While selection of mRNA libraries has generated strong and specific binders against soluble peptides, mRNA display is not compatible for selection against cell-expressed targets. Therefore, there remains a need for methods of selecting against cell-expressed targets such as proteins or protein analogs. Moreover, most prior methods use qPCR to determine the ratio of output to input molecules (percentage recovery) to determine efficiency. However, selected peptides include both specific and non-specific binders. Therefore, methods of selecting only specific binders are needed.

Thus, the present invention provides methods of selecting mRNA display libraries against cell-expressed and soluble peptides or peptide analogs. The present invention also provides a means to monitor enrichment of target-specific peptides in each selection round in a quantitative manner, which would be a more accurate measure of determining efficiency. In addition, the selection conditions of the present invention can be modified based on data obtained in real-time for selection against soluble and cell-expressed targets. Furthermore, the present invention allows for the incorporation of FACS to select target-specific peptides against cell-expressed and recombinant proteins, allows for functional selection (selecting peptides that would have a desired function in a cell-based assay), and gives the ability to identify peptides that internalize within cells.

Puromycin is attached to a short DNA linker to obtain a puromycin-DNA linker. A fluorophore is then fused to the 5'-end of the puromycin-DNA linker such that the resulting DNA-linker is fused to puromycin at its 3'-end, and a fluorophore at its 5'-end. As a result, each mRNA-DNA-peptide molecule in the library is automatically tagged with a fluorophore when annealed to the DNA linker, enabling integration of FACS and flow cytometry with mRNA display platform. In a similar approach, a tag (such as a FLAG^{™} tag) may be incorporated into the mRNA-DNA-peptide complex such that a fluorescent peptide, antibody, or other fluorescent molecule with specificity for the tag binds the mRNA-DNA-peptide molecule, and thus enabling integration of FACS and flow cytometry.

This integration of FACS and flow cytometry provides a means to monitor enrichment of target specific peptides in each selection round in a quantitative manner, whereas prior methods use qPCR to determine the ratio of output to input molecules (percentage recovery). The methods of the present invention provide an advantage over the qPCR percentage recovery determination, which, for the latter, during each selection round both specific and non-specific binders are selected. Therefore, the use of qPCR percentage recovery determination results in the measurement of both specific and non-specific binders in each round of selection. However, enrichment and selection by flow cytometry and FACS allows for selection of mostly specific binders.

The methods of the present invention are also advantageous because selection conditions can be modified based on data obtained in real-time. Furthermore, this approach allows incorporation of FACS to select target-specific peptides against cell-expressed and recombinant proteins, allows for functional selection, and gives the ability to identify peptides that internalize within cells.

Therefore, the present invention provides a method of detecting or selecting for a binding peptide to a target, wherein the method comprises detecting or selecting for an mRNA-DNA-peptide complex attached to at least one fluorophore. In a particular embodiment, the fluorophore is an organic dye, a biological fluorophore, or quantum dots. In a particular embodiment, the detectable substance is an Alexa Fluor. In some embodiments, the detectable substance is conjugated to a puromycin linker.

The present specification also discloses a method of detecting or selecting for a binding peptide to a target, wherein the method comprises detecting or selecting for an mRNA-DNA-peptide complex attached to at least one detectable substance, and wherein the detectable substance is a tag. In an embodiment, the tag comprises a natural or non-natural amino acid. In an embodiment, the tag is a peptide tag. In a particular embodiment, the peptide tag is a FLAG^{™} tag. In an embodiment, the tag is detected by a fluorescent molecule. In a particular embodiment, the tag is detected by a fluorescent peptide. In another particular embodiment, the tag is detected by a fluorescent antibody.

In an embodiment, the methods of the present invention comprise a target immobilized on material that enables sort. In a particular embodiment, the material that enables sort is beads. In an embodiment, the target is a peptide, protein, nucleic acid, sugar, lipid, mammalian cell, or mammalian cell extract. In a particular embodiment, the target is a peptide or protein.

In an embodiment, the methods of the present invention comprise a binding peptide comprising at least one non-natural amino acid.

The binding peptide of the methods of the present invention is detected or selected for by a fluorophore. In other embodiments of the disclosure, the binding peptide is detected or selected for by ELISA, spectrophotometry, fluorescence spectroscopy, or microscopy. In the invention, the binding peptide is detected and selected. In the invention, the binding peptide is detected by flow cytometry and the binding peptide is selected for by FACS.

The methods of the present invention comprise incorporating at least one detectable substance into an mRNA library to produce an mRNA-detectable substance complex. The methods of the present invention comprise translating the mRNA-detectable substance complex to produce an mRNA-peptide-detectable substance complex. The methods of the present invention comprise reverse transcribing the mRNA-peptide-detectable substance complex to obtain an mRNA-DNA-peptide-detectable substance complex. The methods of the present invention comprise incorporating at least one fluorophore into the mRNA library to produce an mRNA-fluorophore complex, translating the mRNA-fluorophore complex to produce an mRNA-peptide-fluorophore complex, and reverse transcribing the mRNA-peptide-fluorophore complex to obtain an mRNA-DNA-peptide-fluorophore complex. The mRNA-DNA-peptide-fluorophore complex is detected by flow cytometry. The mRNA-DNA-peptide-fluorophore complex is selected for by FACS.

In an embodiment, the methods of the present invention comprise transcribing a template DNA library to obtain an mRNA library.

In an embodiment, the methods of the present invention further comprise removing mRNA-DNA-peptide complex not bound to a target.

In an embodiment, the methods of the present invention comprise removing the mRNA-DNA-peptide complex from the target. In a particular embodiment, the mRNA-DNA-peptide complex is removed by the addition of an acid or heat. In a particular embodiment, the mRNA-DNA-peptide complex is removed by heat. In an embodiment, the methods further comprise amplifying by PCR the DNA of the removed mRNA-DNA-peptide complex. In an embodiment, the methods further comprise transcribing the amplified DNA to obtain an mRNA library.

In an embodiment, the methods of the present invention comprise determining the amino acid sequence of the binding peptide. In an embodiment, the methods of the present invention further comprise determining the nucleic acid sequence of the binding peptide.

In an embodiment, the present invention provides the binding peptide detected or selected for by a method of the present invention.

The present disclosure also provides an mRNA-DNA-peptide complex attached to at least one detectable substance. In an embodiment, the present disclosure provides a library of mRNA-DNA-peptide complexes attached to at least one detectable substance. In an embodiment, the detectable substance is a fluorophore. In an embodiment, the fluorophore is an organic dye, a biological fluorophore, or quantum dots. In a particular embodiment, the detectable substance is an Alexa Fluor. In an embodiment, the detectable substance is conjugated to a puromycin linker. In another embodiment, the detectable substance is a tag. In some such embodiments, the tag comprises a natural or non-natural amino acid. In an embodiment, the tag is a peptide tag. In some such embodiments, the tag is a FLAG^{™} tag. In an embodiment, the tag is detected by a fluorescent molecule. In a particular embodiment, the fluorescent molecule is a peptide or antibody attached to a fluorophore.

"Peptides" includes, but is not limited to peptides, polypeptides, proteins, antibodies, antibody fragments, and antibody fusion proteins. "Peptide" may be used interchangeably with polypeptide, protein, antibody, antibody fragment, and antibody fusion protein. A "binding peptide", as used herein, refers to a peptide (including peptide, polypeptide, protein, antibody, antibody fragment, and antibody fusion protein) that binds a target.

"Linker", as used herein, refers to a reagent that can be bound to a translation product. A linker includes, but is not limited to, amino acid, amino acid analog, puromycin, and a puromycin analog. Preferably, the linker is an oligonucleotide fused at its 3' end to an aminonucleoside such as puromycin or a puromycin analog.

"Target", as used herein, refers to a molecule that can interact with a translation product. The target may be a peptide, protein, nucleic acid (DNA or RNA), sugar, lipid, mammalian cell, mammalian cells extract, or another molecule that has the ability to bind to a binding peptide. A target may be purified (including partial purification), inserted into a cell membrane, displayed on phage, displayed on yeast, displayed on baculovirus, or displayed on a cell. A target may also be mammalian cell-expressed protein presented on Nano discs, Amphipols, or liposomes. Nano discs and Amphipols are used to stabilize membrane bound proteins.

A fluorophore attached to (incorporated into) a DNA-linker, or a tag incorporated into the mRNA-DNA-peptide complex, are each an example of a "detectable substance" being incorporated into the mRNA-DNA-peptide complex. "Attach" and "incorporate" (and derivatives thereof) may be used interchangeably herein, as it pertains to a molecule or other such structure containing a detectable substance.

A person of skill in the art can recognize that a FLAG^{™} tag, for example, is given by the peptide sequence DYKDDDK. In addition to a FLAG^{™} tag, examples of other peptide tags that may be used in the methods of the present invention include, but are not limited to, an HA-tag, His-tag, c-myc tag, and S-tag.

A "translation product" refers to the product of mRNA translation. Preferably, the translation product is a peptide that can bind to the target.

"Selection" and derivatives thereof, as used herein, refers to substantially isolating a certain molecule from other molecules in the library. After multiple rounds of selection, it is expected that molecules with the desired phenotype will be enriched in the library.

"Natural amino acid", as used herein, refers to 20 types of amino acids, which are α-aminocarboxylate (or substituted α aminocarboxylate) that are used in standard translation. This include alanine (Ala), Valine (Val), leucine (Leu), isoleucine (Ile), praline (Pro), tryptophan (Trp), phenylalanine (Phe), methionine (Met), glycine (Gly), serine (Ser), threonine (Thr), tyrosine (Tyr), cysteine (Cys), glutamine (Gln), asparagine (Asn), lysine (Lys), arginine (Arg), histidine (His), aspartic acid (Asp), and glutamic acid (Glu).

"Non-natural amino acid", as used herein, refers to an amino acid in general that differs in structure from the 20 types of natural amino acids used during natural translation. A non-natural amino acid may comprise a naturally occurring amino acid having a modified functional group. It includes all non-natural amino acids or artificial amino acids including, but not limited to, D-amino acids, N-methyl amino acids, α-methyl amino acids, N-acyl amino acids, B(beta)-amino acids, Y(gamma)-amino acids, and Ö(delta)-amino acids. Delta amino acids are natural amino acids whose side chain structure is chemically changed/modified in one part, or derivatives having a structure in which the amino group or carboxyl group on the amino acid backbone is substituted. Peptides in which non-natural amino acids are introduced, or as referred to herein "non-natural peptides", include polymers having these various non-natural amino acids as components. Non-natural peptides may be constituted partially or entirely of non-natural amino acids. Hence, non-natural peptides may have a main chain whose structure differs from standard amide bonds.

"Library", as used herein, refers to groups of multiple molecules, such as multiple nucleic acids, translation products, product/linker/mRNA complexes, and translation product/linker/mRNA-cDNA complex molecules. The variety of the library may be expressed as different gene sequences. A DNA or RNA library refers to a DNA library or a RNA library encoding a peptide library.

### EXAMPLES

### Example: Detection of Binding of an mRNA-DNA-Peptide Complex to a Target

A peptide (herein referred to as "Peptide A") attached to its mRNA in an mRNA-DNA-peptide detectable complex is used to determine if flow cytometry can be utilized to detect binding of peptide to its target (herein referred to as "Target A"). Target A may be recombinant protein fused to an AVI-tag (a tag that is fused recombinantly to a peptide of interest to facilitate its site-specific biotinylation *in vivo* by a biotin ligase (BirA) enzyme).

Messenger RNA encoding each peptide is annealed to a fluorophore (e.g. Alexa Fluor 647)-conjugated puromycin linker. Then, in vitro translation (IVT) is initiated using PDPS and mRNA is reverse transcribed, resulting in an mRNA-DNA-peptide A complex. The mRNA-DNA-peptide A complex is added to biotinylated Target A-Avi tag to allow for binding. Streptavidin-coated beads are added to the IVT mix (containing target A-Avi tag) and the complex of streptavidin-coated beads with biotinylated Target A that is bound to the mRNA-DNA-peptide complex is pulled down, washed, and diluted in FACS buffer prior to performing flow cytometry. Streptavidin beads mixed with in vitro transcription translation mix (IVT mix) that lack amino acids and aminoacyl tRNA synthetase (ARSs) [IVT(-)] is used as a negative control to ensure that binding observed by flow cytometry is specific to Target A-Avi tag binding peptides.

Following procedures essentially as described above in this Example, it is expected that strong binding of mRNA-DNA-peptide complexes to its target (Target A) will be detected.

Following procedures essentially as described above in this Example, strong binding of mRNA-DNA-peptide complexes to its target (Target A) was detected.

### Example: Target Specific Enrichment During mRNA Library Selection

To determine if flow cytometry can be used to monitor selection by detecting the enrichment of target-specific peptides, selection rounds against recombinant Target A-Avi conjugated to streptavidin beads can be interrogated.

Chloroacetyl phenylalanine is used to initiate translation and to make a stable thioether bond with a C-terminally engineered cysteine residue. Selection is monitored by determining percent recovery by qPCR. A negative selection against streptavidin beads is incorporated in each round to eliminate non-specific streptavidin binders. An increase in percent recovery is expected to be most noticeable in the later rounds and would suggest enrichment in the population of identified target A binders.

Peptide sequences from later rounds are then determined using Illumina^{®} next generation sequencing. A high frequency of a small number of peptides may demonstrate enrichment of those particular peptides. To determine if the observed increase in percent recovery might be due to selection of non-specific binders, peptide sequences from a later round are compared to peptide sequences from a previous round. It is expected that non-specific binders will be enriched at the later round using the qPCR method. These non-specific binders may include peptides with truncated sequences comprised of one or two amino acids.

Following procedures essentially as described above, a high frequency of a small number of peptides was indicative of enrichment of those particular peptides. Comparison of peptide sequences from a later round to previous round revealed that the highly enriched peptides are truncated sequences comprised of one or two amino acids. In most cases, as the selection rounds increase, non-specific binders will be enriched at the later rounds. qPCR indiscriminately quantifies number of output DNA molecules, therefore true versus non-specific peptides cannot be differentiated using this method. Therefore, other means than qPCR should be employed to improve selection of specific binders.

To follow enrichment of Target A binding peptides from different rounds of selection, flow cytometry is used. DNA from outputs of different rounds is isolated and amplified by PCR to re-constitute each peptide pool with fluorophore-conjugated puromycin linker, and binding of each pool to Target A-AVI by flow cytometry is assessed. Streptavidin plus IVT(-) sample is used for gating and as negative control. Despite the expected increase in percent recovery by qPCR, it is expected that there will be minimal, if any, increase in binding from the different rounds by flow cytometry. This may be, in part, due to the reduced selection of non-specific binders recovered from flow cytometry.

Following procedures essentially as described above, an increase in percent recovery by qPCR suggested that later rounds should bind target much stronger. However, specific binding of the peptide pool to the target by flow cytometry was not observed. Lack of binding of peptide pools to target by flow cytometry corroborated with the enrichment of truncated non-specific peptides in later rounds of selection.

### Example: Use of FACS to Isolate Target Specific Binders From mRNA Library

To determine if a library screened against Target A-AVI can be sorted by FACS, DNA from the output of a later round is transcribed and translated using a puromycin linker fused to a fluorophore, such as Alexa Flour 647, and flow cytometry is performed to detect binding. Gating is around Target A-AVI in complex with streptavidin beads, and it is expected that a low percentage of peptides will bind to Target A (pre-sorted pool). The positive population is sorted by flow cytometry, and binding of the peptide pool from the sorted round to Peptide A-AVI is assessed by flow cytometry. The results are expected to indicate a significant increase in binding of the sorted pool compared to the pre-sorted pool. The results are also expected to demonstrate that selection by FACS results in greater enrichment of Target A binding peptides compared to using the traditional mRNA selection method, and that selection by FACS is an efficient method to eliminate background binders and to isolate specific peptides.

Following procedures essentially as described above, a low percentage of peptides bound to Target A (pre-sorted pool). The positive population was sorted by flow cytometry, and binding of the peptide pool from the sorted round to Target A-AVI was assessed by flow cytometry. The results indicated a significant increase in binding of the sorted pool compared to the pre-sorted pool. The results also demonstrated that selection by FACS resulted in greater enrichment of Target A specific binding peptides compared to using the traditional percent recovery method, and that selection by FACS is an efficient method to eliminate background binders and to isolate specific peptides.

To further determine if selection by percent recovery can result in enrichment of both specific and non-specific binders, the peptide sequences of a sorted round are compared to peptide sequences of corresponding unsorted rounds. It is expected that several of the highest frequency sequences of a sorted round will be different from unsorted rounds. In addition, the frequency of specific peptides is expected to increase after sorting by FACS. This would suggest that selection of mRNA display libraries by FACS using the modified puromycin linker results in highly efficient isolation of peptides with strong binding affinity and specificity.

Following procedures essentially as described above, several of the highest frequency sequences of the peptides from sorted rounds are different from unsorted rounds. The frequency of specific peptides was increased after sorting by FACS. This suggests that selection of mRNA display libraries by FACS using the modified puromycin linker results in highly efficient isolation of peptides with strong binding affinity and specificity.

### Example: Detection and Selection of Cell-Expressed Peptide

The conventional mRNA display selection method cannot efficiently detect cell-expressed peptides because of high noise intrinsic to the system resulting in selection of non-specific peptides. Four rounds of selection are completed using PDPS against a recombinant heterodimeric cell expressed protein (herein referred to as "Protein II"; biotinylated; attached to streptavidin beads). Streptavidin beads are used to pull down complexes of peptide with Protein II and selection is monitored by determining percent recovery in each round. A significant increase in percent recovery was observed at round four (Table 1), and therefore the stringency of selection is increased, in part, by decreasing the concentration of target to favor isolation of stronger binders.

**Table 1. Percent recovery in each round of selection against recombinant Protein II.**

| **Rounds** | **% recovery against biotinylated** |
|---|---|
| | **Protein II** |
| **1** | 0.0024 |
| **2** | 0.0072 |
| **3** | 0.27 |
| **4** | 2.92 |
| **5** | 0.057 |

To determine if a cell-expressed protein can be used as a target by the methods of the present invention, flow cytometry is used against either recombinant or cell-expressed Protein II to monitor enrichment of Protein II-binders during selection. DNA from output of rounds two to five is transcribed, annealed to modified puromycin linker, and translated to constitute a fluorophore library for each round. Binding of each mRNA-DNA-peptide pool to recombinant or cell-expressed Protein II is detected by flow cytometry.

Following procedures essentially as described above, the outputs of round two to round five bound strongly to both recombinant and cell-expressed Protein II as detected by flow cytometry (Table 2). These data demonstrate that the methods of the present invention can be used to select for both recombinant and cell-expressed peptides.

**Table 2. Binding of mRNA-DNA-peptide library to Protein II from different rounds of selection and detected by flow cytometry.**

| | **% MFI** | |
|---|---|---|
| **Rounds** | **Recombinant Protein II** | **Cell-Expressed Protein II** |
| **2** | 6.48 | 25.7 |
| **3** | 77.4 | 46.1 |
| **4** | 98 | 53.7 |
| **5** | 99.9 | 64.3 |
| **IVT(-)** | 0.062 | 0.91 |

The DNA from output of round two is then used to sort Protein II binders using FACS. Since a recombinant form of the protein is used in the first round of selection, sorting is performed with Protein II expressed on CHO cells to isolate macrocycles that bind to both forms (recombinant or cell-expressed) of the target. To determine if sorting by FACS results in enrichment of Protein II binders, binding of pre- and post-sorted peptide pools to Protein II is examined. The results indicate enrichment in the population of specific binders as determined by flow cytometry post-sort (4% binders in pre-sort compared to 40% post-sort). Binding of the sorted pool to parental CHO cells that lack expression of Protein II was minimal. Next generation sequencing of round two (sorted) peptides revealed that new families, whose sequences were not present at detectable levels in pre-sort and later rounds of selections (that was carried out by conventional mRNA display selection methods), had emerged.

To determine how binding and functional activity of the most enriched peptides from the round two (sorted) pool compare to round five, peptides identified from round two (sorted) pool will be chemically synthesized and tested in a cell-based functional assay to determine if binding of Protein II with its ligand can be blocked by the sorted peptides. The data are expected to demonstrate that peptides present in round two (sorted) pool will inhibit the interaction of Protein II with its ligand. These results would indicate that FACS sorting in the earlier rounds of selection results in isolation and identification of peptides with functional activity that might be depleted from the library in later rounds due to carrying out multiple rounds of selection.

Following procedures essentially as described above, peptides present in round two (sorted) pool inhibited the interaction of Protein II with its ligand.

### Example: Design and Optimization of DNA-Linker Conjugated to Fluorophore

Fluorophores are conjugated (attached) to the 5'-end of puromycin linkers to enable detection of mRNA-DNA-peptide complex by flow cytometry. Fluorophores are either directly conjugated to the oligo at its 5'-end using a NHS Ester linker or a spacer of varying lengths. Attachment may occur, for example, by covalent linkage of puromycin to the 3'-end of the short oligo linker via multiple hexa-ethylene glycol spacer. It is thought that increasing the length of the spacer is most effective when multi-pass membrane proteins (GPCRs and ion-channels) are the targets or when the peptides are binding to a cavity or groove of a protein. For example, binding of an mRNA-DNA-peptide to a multi-span cell expressed protein (herein referred to as "Protein III") complex, expressed on the cell surface of HEK cells, is better detected by flow cytometry as the spacer length is increased (Table 3). This might be due to enhanced accessibility of the fluorophore with the increased linker length. However, the data in Table 3 also demonstrate that a spacer might not be required for detection by flow cytometry.

**Table 3. Detection of binding of mRNA-DNA-Protein III complex using modified puromycin linker.**

| | % MFI | |
|---|---|---|
| **Spacer** | **Receptor-HEK** | **HEK*** |
| No spacer (direct conjugation of fluorophore to oligo) | 73% | 5% |
| +C9 | 94% | 4% |
| Triethylene glycol spacer of MW of 212 Da | | |
| +C18 | 96% | 3% |
| 18-atom hexa-ethyleneglycol spacer of MW 344.3 Da | | |

| | | |
|---|---|---|
| * HEK cells without exogenous receptor is used as a negative control. | | |

## Claims

1. A method of detecting and selecting for a binding peptide to a target, wherein the method comprises:
(a) incorporating at least one fluorophore into an mRNA library to produce an mRNA-fluorophore complex;
(b) translating the mRNA-fluorophore complex to produce an mRNA-peptide-fluorophore complex;
(c) reverse transcribing the mRNA-peptide-fluorophore complex to obtain an mRNA-DNA-peptide-fluorophore complex;
(d) detecting the mRNA-DNA-peptide-fluorophore complex by flow cytometry; and
(e) selecting the binding peptide by fluorescence activated cell sorting.

2. The method of claim 1, wherein the fluorophore is conjugated to a puromycin linker.

3. The method of claim 2, wherein the fluorophore is an organic dye, a biological fluorophore, or quantum dots.

4. The method of any one of claims 1 to 3, wherein the binding peptide comprises at least one non-natural amino acid.

5. The method of any one of claims 1 to 4, wherein the method further comprises transcribing a template DNA library to obtain an mRNA library.

6. The method of any one of claims 1 to 5, further comprising removing mRNA-DNA-peptide complex not bound to a target.

7. The method of any one of claims 1 to 6, further comprising removing mRNA-DNA-peptide complex from a target.

8. The method of claim 7, wherein the mRNA-DNA-peptide complex is removed by heat.

9. The method of claim 6 or claim 7, further comprising amplifying by PCR the DNA of the removed mRNA-DNA-peptide complex.

10. The method of claim 9, further comprising transcribing the DNA to obtain an mRNA library.

11. The method of any one of claims 1 to 10, further comprising determining the amino acid sequence of the binding peptide.

12. The method of any one of claims 1 to 10, further comprising determining the nucleic acid sequence of the binding peptide.

## Patentansprüche

1. Verfahren zum Erkennen und Auswählen eines bindenden Peptids an ein Ziel, wobei das Verfahren umfasst:
(a) Einbringen mindestens eines Fluorophors in eine mRNA-Bibliothek, um einen mRNA-Fluorophor-Komplex zu erzeugen;
(b) Umsetzen des mRNA-Fluorophor-Komplexes, um einen mRNA-Peptid-Fluorophor-Komplex zu erzeugen;
(c) reverses Transkribieren des mRNA-Peptid-Fluorophor-Komplexes, um einen mRNA-DNA-Peptid-Fluorophor-Komplex zu erhalten;
(d) Erkennen des mRNA-DNA-Peptid-Fluorophor-Komplexes durch Durchflusszytometrie; und
(e) Auswählen des bindenden Peptids durch fluoreszenzaktivierte Zellsortierung.

2. Verfahren nach Anspruch 1, wobei das Fluorophor an einen Puromycin-Linker konjugiert ist.

3. Verfahren nach Anspruch 2, wobei das Fluorophor ein organischer Farbstoff, ein biologisches Fluorophor oder Quantenpunkte ist.

4. Verfahren nach einem der Ansprüche 1 bis 3, wobei das bindende Peptid mindestens eine nicht natürliche Aminosäure umfasst.

5. Verfahren nach einem der Ansprüche 1 bis 4, wobei das Verfahren ferner das Transkribieren einer DNA-Vorlagenbibliothek umfasst, um eine mRNA-Bibliothek zu erhalten.

6. Verfahren nach einem der Ansprüche 1 bis 5, ferner umfassend ein Entfernen des mRNA-DNA-Peptid-Komplexes, der nicht an ein Ziel gebunden ist.

7. Verfahren nach einem der Ansprüche 1 bis 6, ferner umfassend das Entfernen des mRNA-DNA-Peptid-Komplexes von einem Ziel.

8. Verfahren nach Anspruch 7, wobei der mRNA-DNA-Peptid-Komplex durch Hitze entfernt wird.

9. Verfahren nach Anspruch 6 oder 7, ferner umfassend ein Amplifizieren, durch PCR, der DNA des entfernten mRNA-DNA-Peptid-Komplexes.

10. Verfahren nach Anspruch 9, ferner umfassend das Transkribieren der DNA, um eine mRNA-Bibliothek zu erhalten.

11. Verfahren nach einem der Ansprüche 1 bis 10, ferner umfassend ein Bestimmen der Aminosäuresequenz des bindenden Peptids.

12. Verfahren nach einem der Ansprüche 1 bis 10, ferner umfassend das Bestimmen der Nukleinsäuresequenz des bindenden Peptids.

## Revendications

1. Procédé de détection et de sélection d'un peptide de liaison à une cible, dans lequel le procédé comprend :
(a) l'incorporation d'au moins un fluorophore dans une bibliothèque d'ARNm pour produire un complexe de fluorophore ARNm ;
(b) la translation du complexe de fluorophore ARNm pour produire un complexe de fluorophore-peptide ARNm ;
(c) la transcription inverse du complexe de fluorophore-peptide ARNm pour obtenir un complexe de fluorophore-peptide ARNm ;
(d) la détection du complexe de fluorophore-peptide ARNm-AND par cytométrie de flux ; et
(e) la sélection du peptide de liaison par tri cellulaire activé par fluorescence.

2. Procédé selon la revendication 1, dans lequel le fluorophore est conjugué à un lien de puromycine.

3. Procédé selon la revendication 2, dans lequel le fluorophore est un colorant organique, un fluorophore biologique ou des points quantiques.

4. Procédé selon l'une quelconque des revendications 1 à 3, dans lequel le peptide de liaison comprend au moins un acide aminé non naturel.

5. Procédé selon l'une quelconque des revendications 1 à 4, dans lequel le procédé comprend en outre la transcription d'une bibliothèque d'ADN modèle pour obtenir une bibliothèque d'ARNm.

6. Procédé selon l'une quelconque des revendications 1 à 5, comprenant en outre l'élimination du complexe de peptide ARNm-ADN non lié à une cible.

7. Procédé selon l'une quelconque des revendications 1 à 6, comprenant en outre l'élimination du complexe de peptide ARNm-ADN d'une cible.

8. Procédé selon la revendication 7, dans lequel le complexe de peptide ARNm-ADN est éliminé par la chaleur.

9. Procédé selon la revendication 6 ou la revendication 7, comprenant en outre l'amplification par PCR de l'ADN du complexe de peptide ARNm-ADN éliminé.

10. Procédé selon la revendication 9, comprenant en outre la transcription de l'ADN pour obtenir une bibliothèque d'ARNm.

11. Procédé selon l'une quelconque des revendications 1 à 10, comprenant en outre la détermination de la séquence d'acides aminés du peptide de liaison.

12. Procédé selon l'une quelconque des revendications 1 à 10, comprenant en outre la détermination de la séquence d'acide nucléique du peptide de liaison.
